Europäisches Patentamt

European Patent Office

Office européen des brevets

(11) Publication number: **0 297 216 B1**

# EUROPEAN PATENT SPECIFICATION

(45) Date of publication of patent specification: **18.03.92**   (51) Int. Cl.⁵: **A61M 1/36**, B04B 7/00

(21) Application number: **88104480.4**

(22) Date of filing: **21.03.88**

(54) **Centrifugation bowl for the continuous centrifugation of blood.**

(30) Priority: **08.04.87 IT 2003887**

(43) Date of publication of application:
**04.01.89 Bulletin 89/01**

(45) Publication of the grant of the patent:
**18.03.92 Bulletin 92/12**

(84) Designated Contracting States:
**CH DE ES FR GB IT LI SE**

(56) References cited:
**US-A- 4 140 268**
**US-A- 4 668 214**

(73) Proprietor: **DIDECO S.p.A.**
**Via Galilei 3**
**I-41037 Mirandola (Province of Modena)(IT)**

(72) Inventor: **Panzani, Ivo**
**Via Posta, 58**
**I-41037 Mirandola (Province of Modena)(IT)**

(74) Representative: **Modiano, Guido et al**
**MODIANO, JOSIF, PISANTY & STAUB**
**Modiano & Associati Via Meravigli, 16**
**I-20123 Milano(IT)**

Rank Xerox (UK) Business Services

## Description

It is known that blood centrifugation to achieve separation of the red corpuscles from the other components, such as plasma, white corpuscles and platelets, or, as sometimes occurs, from physiological solution in which said red corpuscles are contained, is currently performed in devices known as cells, which comprise an outer container which is truncated cone-shaped or, idiomatically, of bell-shaped, and delimits a space portion accomodating a body which is rigidly connected with and coaxial to the container and is formed by two revolution walls connected at their end edges: a wall facing the outer container and having a shape substantially corresponding to the truncated-cone shape of the latter, and substantially cylindrical inner wall; no access of fluid is provided within the body described, which is hermetically sealed.

The outer body, with the body rigidly connected therewith, is intended to be gripped by a rotating mandrel for rotating therewith, and carries at its top, through suitable gaskets and seals, a stationary joint which comprises two conduits which are coaxial at least in the upper region and are provided, at the upper end, with connection portions for connecting to tubes leading to other devices: an inner conduit which extends in the space portion delimited by the inner, substantially cylindrical wall of the body rigidly connected with the outer container and ends close to the bottom of said container, and an outer conduit which ends, at the lower portion, into a gap comprised between two facing discs positioned at the base of the stationary joint, that is, in the space portion at the top of the body rigidly connected with the outer body.

In these known cells, the whole blood is fed into the cell through the inner conduit and reaches the bottom of the outer container where it is subject to a centrifugal force: as a consequence thereof, the red corpuscles, which are heavier, collect and concentrate against the wall of the outer container, separated at a substantially vertical front from the lighter fractions, composed of plasma, platelets, and white corpuscles, which remain inwards.

In the course of time, continuous inflow of whole blood causes the level of the separated components to rise in the cell, and at a certain point the light components, that is plasma, platelets and white corpuscles, begin to enter the gap comprised between the two discs of the stationary joint which are placed at the base of the latter, then travel along the outer conduit which indeed ends at this gap, and are evacuated.

The process goes on until continuous increase of concentrated red corpuscles in the cell causes the separation front between corpuscles and light components to reach the gap between the discs of the stationary joint, and at this point it is obvious that the process must be stopped to prevent outflow from the cell also of red corpuscles.

Access of whole blood is then interrupted and the mandrel which rotates the cell is stopped; at this point, the cell is full of concentrated red corpuscles which can be sucked through means of the central conduit to free the cell and to be sent to the intended uses.

The operation is fully similar when red corpuscles in physiological solution are to be separated from the solution itself.

From the foregoing, it is evident that a disadvantageous feature of known cells consists in the fact that extraction of the concentrated red corpuscles therefrom is possible only when the corpuscles have completely filled the bell, and therefore only after a remarkable amount of blood has been fed thereto.

This disadvantage is particularly relevant in case of intraoperative auto-transfusion: that is recovery of blood spilled by a patient during surgery, which is sucked and sent to a cell where washing with physiological solution is performed, together with separation of concentrated red corpuscles, which it is vitally important to rapidly reinfuse to the patient.

With known cells, this rapid reinfusion is clearly impossible, since it is necessary, as mentioned above, for the cell to be completely filled with red corpuscles in order to stop the cell and extract red corpuscles, and use of small-volume cells certainly does not solve the problem, since it is clearly impossible to have a range of dimensions such as to optimize performance in the great variety of actual cases. US-A-4 140 268 shows a centrifugation device as defined in the pre-characterizing portion of appended claim 1.

Another disadvantageous feature of known cells is due to their discontinuous operation, and envolves idle times which are clearly not appreciated since they extend the overall time of the operation.

The aim of the present invention is therefore to provide a continuous blood centrifugation cell which allows extraction of the concentrated blood corpuscles without waiting for the same to completely fill the cell, and which furthermore achieves a continuous operation, with complete elimination of idle times.

Within the proposed aim, an object of the invention is to provide a cell having a particularly simple structure, such as to ensure a modest cost and the maximum reliability in operation.

The above aim and object are achieved by a continuous blood centrifugation cell, according to the invention as defined in the appended claims.

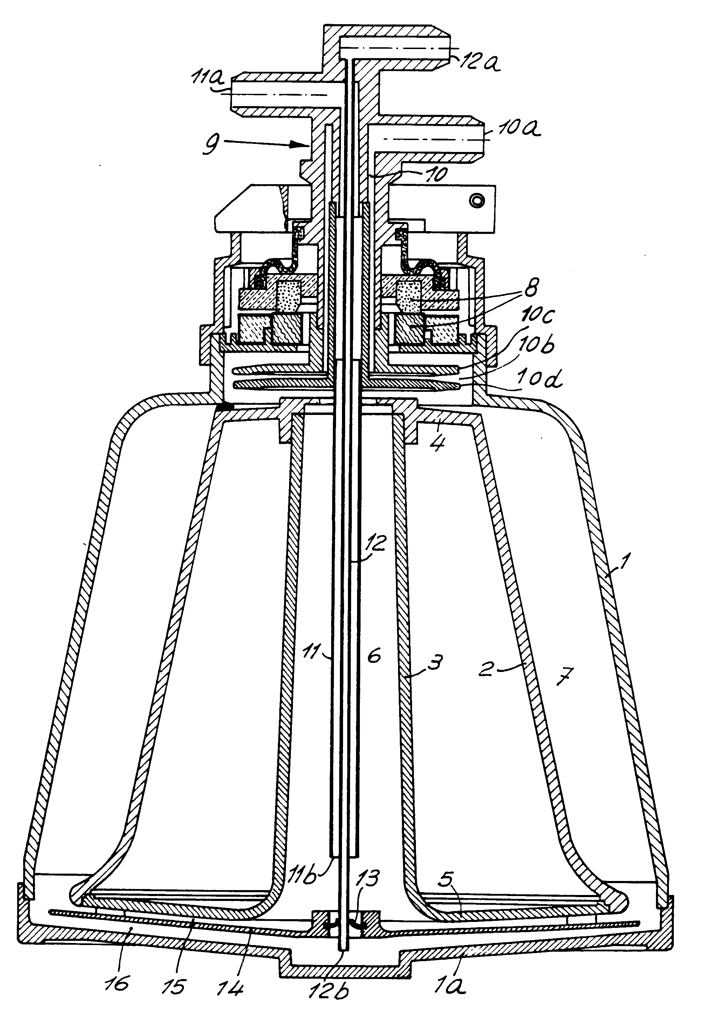

Further features and advantages of the invention will become apparent from the description of a preferred, but not exclusive, embodiment of the invention, illustrated only by way of non-limitative example in the accompanying drawing, the only figure whereof is a cross section view along a plane extending through the rotation axis.

With reference to said figure, 1 indicates the outer bell-shaped container having a bottom 1a and delimiting a space portion accomodating a body which is coaxial and rigidly connected with the container 1 and is formed by revolution walls 2 and 3 connected at the end edges by walls 4 and 5; 6 indicates the space portion delimited by the inner wall 3, and 7 indicates the space portion comprised between the outer wall 2 of the body and the wall of the outer container 1. The outer container 1 is intended to be rotated, together with the described body rigidly connected therewith, by a mandrel not illustrated in the figure, and carries at its top, through suitable per se known gaskets and sealing rings generally indicated at 8, a stationary joint generally indicated at 9.

This joint comprises three conduits which are coaxial to the rotation axis of the cell, and all are provided with connections for coupling to tubes leading to other devices, which conduits are now described in detail.

A first conduit is an outer conduit 10 having upwardly a connection portion 10a and opening, at the lower end thereof, into a gap 10b comprised between two facing discs 10c, 10d rigidly connected with the stationary joint.

A second conduit is an intermediate conduit 11, having a connection portion 11a and downwardly opening at 11b within the space portion 6.

A third conduit is an inner conduit 12, having a connection portion 12a and extending through a sealing gasket 13 in a hole provided in a partition wall 14, which is rigidly connected with the wall 5 and defines two superimposed passages 15 and 16 peripherally communicating with each other, the inner conduit 12 ending at 12b within the passage 16.

The operation of the invention is now described with reference to separation of red corpuscles from whole blood, with the light fraction being essentially composed of plasma, platelets and white corpuscles, but an identical operation is carried out when the red corpuscles are separated from physiological solution.

Through the conduit 11, whole blood is continuously fed into the cell, which is rotated; as soon as it flows out from opening 11b, it is subject to a centrifugal force and enters the passage 15; the red corpuscles concentrate against the wall of the outer container 1, separated at a substantially vertical front from the light fractions which remain inwards and which, at a certain point, as the inflow of whole blood continues, reach the gap 10b and travel along the outlet conduit 10; all this according to known methods.

Contrary to known cells, it is however possible, in any moment, to extract red corpuscles, irrespective of the amount thereof in the cell, and without stopping inflow of blood: it is indeed sufficient to apply a vacuum or negative pressure in the internal tube 12 to cause flow of red corpuscles through the passage 16, which peripherally communicates with the passage 15 in a region where only red corpuscles are present, until these corpuscles enter the tube 12 through the end 12b.

The position of the separation front between red corpuscles and light fractions, which obviously shifts during the operation according to the mutual variations of rates of inflow of whole blood and of outflow of red corpuscles, is controlled either visually by an operator, by virtue of transparency of the cell walls, or by means of photocells, to ensure the maximum operation correctness.

With the cell according to the invention it is thus possible to fully achieve the proposed aims: e.g. in case of auto-transfusion, after a certain, even small, amount of blood has been recovered and sent to the cell, it is possible to reinfuse the patient with the red corpuscles separated from the even small amount of blood with great timeliness.

The great increase in effectiveness of the cell according to the invention due to elimination of idle times by virtue of possible continuous operation, is furthermore evident.

The invention described is susceptible to numerous modifications and variations, all within the inventive concept; thus, for example, the passage 16 may be formed by a plurality of small radial channels. In the manufacture of the invention, all the details may be replaced with technically equivalent elements; furthermore, the employed materials, as well as the dimensions and the shapes, may be any according to the requirements.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. A continuous blood centrifugation cell, comprising an outer rotatable container (1) delimiting a first space portion (7) accommodating a body which is rigidly connected with and coaxial to said container (1) and is formed by inner and outer revolution walls (2,3) connected at their upper and lower end edges, the container (1) and the body being arranged such as to form a second space portion (6) delimited by the inner revolution wall (3) of said body and a third space portion comprised between the bottom of the outer container (1) and the said body, said outer container (1) carrying, at the top portion thereof, a stationary joint (9) comprising conduits (10,11,12) which have, at upper ends thereof, connection portions (10a,11a,12a) and are coaxial to said joint (9), an outer conduit (10) being connected to a first connection portion (10a) and ending at a gap (10b) located at the base of said stationary joint (9) in communication with the first space portion (7) defined outside the outer wall (2) of said rigidly connected body, an intermediate conduit (11) being connected to a second connection portion (11a), coaxially protruding from the stationary joint (9) and being in communication with the second space portion (6), characterized in that the third space portion is divided by a partition wall (14) into two superimposed passages (15,16) which communicate peripherally, the upper one whereof opens onto the second space portion (6), said partition wall (14) having a hole for the passage, through a sealing gasket (13), of an inner conduit (12) coaxially protruding from said stationary joint (9), connected to a third connecting portion (12a) and extending down to the bottom of the outer container (1), within the lower one of said superimposed passages (15,16).

2. A cell according to claim 1, characterized in that the lower passage (16) comprises a plurality of small radial channels.

**Revendications**

1. Cellule pour la centrifugation continue du sang, comprenant un conteneur rotatif externe (1) délimitant une première portion d'espace (7) recevant un corps qui est rigidement relié audit conteneur (1) et coaxial à celui-ci, et est formé par des parois de révolution interne et externe (3, 2), reliées à leurs bords d'extrémité inférieurs et supérieurs, le conteneur (1) et le corps étant agencés de façon à former une seconde portion d'espace (6) délimitée par la paroi interne de révolution (3) dudit corps et une troisième portion d'espace comprise entre le fond du conteneur externe (1) et ledit corps, ledit conteneur externe (1) portant, à sa partie d'extrémité supérieure, un raccord fixe (9) comprenant des conduits (10,11,12) qui présentent, à leurs extrémités supérieures, des parties de liaison (10a,11a,12a) et sont coaxiaux audit raccord (9), un conduit externe (10) étant relié à une première partie de liaison (10a) et s'achevant au niveau d'un espace (10b) situé à la base dudit raccord fixe (9) en communication avec la première portion d'espace (7) définie extérieurement à la paroi externe (2) dudit corps rigidement relié, un conduit intermédiaire (11) étant relié à une seconde partie de liaison (11a), faisant saillie coaxialement du raccord fixe (9) et étant en communication avec la seconde portion d'espace (6), caractérisée en ce que la troisième portion d'espace est divisée par une paroi de séparation (14) en deux passages superposés (15,16) qui communiquent périphériquement, dont le premier de ceux-ci débouche dans la seconde portion d'espace (6), ladite paroi de séparation (14) présentant un trou pour le passage, à travers un joint d'étanchéité (13), d'un conduit interne (12) faisant saillie coaxialement du raccord fixe (9), relié à une troisième partie de liaison (12a) et s'étendant vers le fond du conteneur externe (1), à l'intérieur du plus bas desdits passages superposés (15,16).

2. Cellule selon la revendication 1, caractérisée en ce que le passage inférieur (16) comprend une pluralité de petits canaux radiaux.

**Patentansprüche**

1. Zelle zum kontinuierlichen Schleudern von Blut, enthaltend einen äußeren drehbaren Behälter (1), der einen ersten ein Gehäuse aufnehmenden Raumbereich (7) begrenzt, wobei das Gehäuse koaxial und feststehend mit dem Behälter (1) verbunden ist und innere und äußere umlaufende Wände (2, 3) aufweist, die an den oberen und unteren Kanten miteinander verbunden sind, wobei der Behälter (1) und das Gehäuse so angeordnet sind, daß ein zweiter Raumbereich (6) gebildet wird, der durch die innere umlaufende Wand (3) des Gehäuses und einen dritten Raumbereich begrenzt wird, der zwischen dem Boden des äußeren Behälters (1) und dem Gehäuse enthalten ist, wobei der äußere Behälter (1) in seinem oberen Bereich eine feststehende Verbindung (9) trägt, die Leitungen (10, 11, 12) umfaßt, die an ihren oberen Enden Verbindungsbereiche (10a, 11a, 12a) aufweisen und koaxial zur Verbindung (9) angeordnet sind, wobei eine äußere Leitung (10) mit einem ersten Verbindungsbereich (10a) verbunden ist und in einem Spalt (10b) an der Basis der feststehenden Verbindung (9) in Verbindung mit dem ersten Raumbereich (7) endet, der außerhalb der äußeren Wand (2) des feststehend verbundenen Gehäuses vorgesehen ist, wobei eine mittlere Leitung (11) mit einem zweiten Verbindungsbereich (11a) verbunden ist, koaxial aus der feststehenden Verbindung (9) hervorsteht und mit dem zweiten Raumbereich (6) in Verbindung steht, dadurch gekennzeichnet, daß der dritte Raumbereich durch eine Trennwand (14) in zwei übereinanderliegende Durchgänge (15, 16) geteilt ist, die am Umfang miteinander in Verbindung stehen, wobei der obere der beiden zum zweiten Raumbereich (6) hin öffnet, wobei die Trennwand (14) ein Loch für die Durchführung einer inneren Leitung (12) über einen Dichtungsring (13) aufweist, wobei die Leitung (12) koaxial aus der feststehenden Verbindung (9) hervorsteht, mit einem dritten Verbindungsbereich (12a) verbunden ist und nach unten zum Boden des äußeren Behälters (1) innerhalb des unteren der übereinanderliegenden Durchgängen (15, 16) reicht.

2. Zelle nach Anspruch 1, dadurch gekennzeichnet, dass der untere Durchgang (16) eine Vielzahl von radialen Kanälen aufweist.